# EUROPEAN PATENT SPECIFICATION

(11) **EP 3 157 561 B1**
(45) Date of publication and mention of the grant of the patent: **18.12.2019**
(21) Application number: 15809699.0
(22) Date of filing: 17.06.2015
(51) Int. Cl.: A61K 39/395

(54) **IMPROVED ALPHA-V BETA-8 ANTIBODIES**
VERBESSERTE ALPHA-V BETA-8-ANTIKÖRPER
ANTICORPS ALPHA-V BÊTA-8 AMÉLIORÉS

(30) Priority: 17.06.2014 US 201462013114 P
(43) Date of publication of application: 26.04.2017
(73) Proprietor: MedImmune Limited, Cambridge CB21 6GH (GB); The Regents of the University of California, Oakland, CA 94607 (US)
(72) Inventor: NISHIMURA, Stephen, San Francisco California 94110 (US); LOU, Jianlong, San Francisco California 94110 (US); CORMIER, Anthony, San Francisco California 94110 (US); BARON, Jody Lynn, San Francisco California 94143 (US); MARKS, James D., San Francisco California 94110 (US); MURRAY, Lynne, Cambridge Cambridgeshire CB21 6GH (GB); TSUI, Ping, Gaithersburg Maryland 20878 (US); WU, Yanli, Gaithersburg Maryland 20878 (US)
(74) Representative: AstraZeneca
(86) International application number: PCT/US2015/036284
(87) International publication number: WO 2015/195835

(56) References cited:
- WO-A1-2006/099875
- WO-A2-2008/105886
- WO-A2-2013/026004
- WO-A2-2013/026004
- US-A1- 2012 058 128
- MINAWAGA, S ET AL.: 'Selective Targeting Of TGF-Beta Activation To Treat Fibroinflammatory Airway Disease.' SCI TRANSL MED. vol. 6, no. 241, 18 June 2014, pages 1 - 14, XP009182474 DOI: :10.1126/SCITRANSLMED.3008074

## Description

### CROSS-REFERENCE TO RELATED APPLICATIONS

This application claims to the benefit of U.S. Patent Application Serial No. 62/013,114, filed June 17, 2014.

### BACKGROUND

The multifunctional cytokine transforming growth factor-β (TGF-β) affects immune, endothelial, epithelial, and mesenchymal cells during development and adult life in invertebrate and vertebrate species. TGF-β plays a role in T-cell, cardiac, lung, vascular, and palate development. Mice deficient in TGF-β1 either die in utero, owing to defects in yolk sac vasculogenesis, or survive to adulthood with severe multiorgan autoimmunity. Genetic deletion of TGF-β signaling mediator Smad2 reveals that it is essential in early patterning and mesodermal formation. Mice lacking Smad3 are viable and fertile, but exhibit limb malformations, immune dysregulation, colitis, colon carcinomas, and alveolar enlargement. In adult tissues, the TGF-β pathway is involved in the immune, mesenchymal, and epithelial cell interactions to maintain homeostasis in response to environmental stress.

The homeostatic pathways mediated by TGF-β are perturbed in response to chronic repetitive injury. TGF-β is a major profibrogenic cytokine in response to injury, delaying epithelial wound healing. TGF-β inhibits epithelial proliferation and migration, promotes apoptosis, and expands the mesenchymal compartment by inducing fibroblast recruitment, fibroblast contractility, and extracellular matrix deposition. Intratracheal transfer of adenoviral recombinant TGF-β1 to the rodent lung dramatically increases fibroblast accumulation and expression of type I and type III collagen around airways and in the pulmonary interstitium. Neutralizing anti-TGF-β antibodies can block bleomycin or radiation-induced pulmonary fibrosis.

Increased TGF-β activity can play a role in fibrotic lung disease, glomerulosclerosis, and restenosis of cardiac vessels, primarily mediated by TGF-β1. TGF-β1 function in humans is complex, as indicated by hereditary disorders involving either TGF-β1 itself or its signaling effectors. Mutations that increase the activity of the TGF-β pathway lead to defects in bone metabolism (*ie,* Camurati-Engelmann disease), in connective tissue (*ie,* Marfan syndrome), and in aortic aneurysms (*ie,* Loeys-Dietz syndrome). Mutations that lead to decreased activity of the TGF-β pathway correlate with cancer. The role of TGF-β as a tumor suppressor in cancer is not straightforward, however, because TGF-β can also enhance tumor growth and metastasis.

Despite the multiple essential functions of TGF-β, a single dose or short-term administration of a pan-TGF-β neutralizing antibody is well tolerated. No side effects are observed in rodents at doses that inhibit organ fibrosis or carcinoma cell growth and metastasis. This treatment also effectively inhibits experimental fibrosis. Single-dose phase I/II clinical trials using neutralizing pan-TGF-β antibodies are ongoing for metastatic renal cell carcinoma, melanoma, focal segmental glomerulosclerosis, and idiopathic pulmonary fibrosis.

Some TGF-β isoforms are expressed ubiquitously in mammals (TGF-β 1-3), but are maintained in an inactive form by non-covalent interaction with a propeptide, the latency associated domain of TGF-β (LAP). For TGFβ to signal, it must be released from its inactive complex by a process called TGFβ activation. The latent TGF complex includes 3 components: the active (mature) TGFβ dimmer, LAP (latency associated peptide) and LTBP (latent TGFβ binding protein). LAP is a dimer, linked by two disulfide bonds, that represents the N-terminal end of the TGFβ precursor protein. The mature TGFβ protein represents the C terminal end of the precursor, and forms a disulfide-linked dimer of about 25kD. The bond between TGFβ and LAP is proteolytically cleaved within the Golgi, but the TGF-β propeptide remains bound to TGFβ by non-covalent interactions. The complex of TGFβ and LAP is called the small latent complex (SLC). It is the association of LAP and TGFβ that confers latency. LAP-TGFβ binding is reversible and the isolated purified components can recombine to form an inactive SLC. Both the SLC and the larger complex are referred to herein as latent TGF-β, as both are inactive.

In general, integrins are adhesion molecules and mediate the attachment of cells to extracellular matrix proteins. Integrin αvβ8 binds to the LAP of TGF-β and mediates the activation of TGF-β1 and 3 (Mu et al. (2002) J. Cell Biol. 159:493). Integrin αvβ8-mediated activation of TGF-β is required for *in vivo* activation of TGF-β (*i.e*., release of the mature TGF-β polypeptide), thus αvβ8 is a gatekeeper of TGF-β function. Integrin αvβ8 is expressed in normal epithelia (*e.g*., airway epithelia), mesenchymal cells, and neuronal tissues. Integrin αvβ8-mediated activation of TGF-β can result in COPD, pulmonary fibrosis, arthritis, inflammatory bowel disease, hepatic and renal fibrosis, inflammatory brain autoimmune diseases and demyelinating diseases(*e.g*., MS, transverse myelitis, Devic's disease, Guillain-Barré syndrome), neuroinflammation, kidney disease, and cancer growth and metastasis (*see*, *e.g.*, WO2013/026004).

### BRIEF SUMMARY

Provided herein are antibodies (*e.g*., monoclonal, recombinant, and/or chemically modified) that specifically bind integrin αvβ8 such that binding of the antibody inhibits release of active mature TGFβ peptide, but does not significantly inhibit adhesion of latent TGFβ to αvβ8 on a αvβ8-expressing cell, wherein the antibody does not include the heavy chain CDR2 sequence of the 37E1B5 antibody (*e.g*., SEQ ID NO:7). In some aspects, the antibody binds an epitope in the head and/or hybrid domains of β8, and upon binding, causes a conformational change in β8 that reduces the angle between the head and hybrid domains of β8 compared to β8 not contacted with the antibody. In some aspects, the antibody binds an epitope that includes the sequence of SEQ ID NO:16. In some aspects, at least one amino acid in a CDR of the antibody is glycosylated. In some aspects, the CDR selected from the light chain CDR1, CDR2, CDR3, and the heavy chain CDR1, CDR2, CDR3. The CDRs can be determined according to any known method, *e.g*., Kabat, Chothia, IMGT, or AbM. In some aspects, the glycosylated amino acid is in the heavy chain CDR2 of the antibody. In some aspects, the glycosylated amino acid is at a position corresponding to amino acid position 10 in any one of SEQ ID NOs:1-6.

Further provided are antibodies (*e.g*., monoclonal, recombinant, and/or chemically modified) that specifically bind integrin αvβ8 such that binding of the antibody inhibits release of active mature TGFβ peptide, but does not significantly inhibit adhesion of latent TGFβ to αvβ8 on a αvβ8-expressing cell, wherein the antibody binds an epitope in the head and/or hybrid domains of β8, and upon binding, causes a conformational change in β8 that reduces the angle between the head and hybrid domains of β8 compared to β8 not contacted with the antibody; and wherein the antibody is modified to comprise a heterologous glycosylated amino acid in at least one amino acid in a CDR of the antibody. The CDRs can be determined according to any known method, *e.g*., Kabat, Chothia, IMGT, or AbM. In some aspects, the CDR selected from the light chain CDR1, CDR2, CDR3, and the heavy chain CDR1, CDR2, CDR3. In some aspects, the glycosylated amino acid is in the heavy chain CDR2 of the antibody. In some aspects, the glycosylated amino acid is at a position corresponding to amino acid position 10 in any one of SEQ ID NOs:1-6. In some aspects, the antibody binds an epitope that includes the sequence of SEQ ID NO:16. In some aspects, the antibody does not comprise the heavy chain CDR2 sequence of SEQ ID NO:7.

In some aspects, the antibody, upon binding β8, reduces the angle between the head and hybrid domains of β8 by at least 5° (*e.g.,* at least 6°, 7°, 8°, 9°, 10°, 11°, 12° or more) compared to β8 not contacted with the antibody, or compared to an antibody lacking a glycan in a CDR (*e.g*., heavy chain CDR2).

In some aspects, the antibody comprises the heavy and light chain CDR sequences of a 14E5 antibody or variant thereof (*e.g*., 2A8, 2A10, 2C6), wherein at least one amino acid in a CDR is modified to introduce a glycosylated amino acid. In some aspects, the antibody comprises the heavy and light chain CDR sequences of a 11E8 antibody or variant thereof (*e.g*., 2B8, 2A4), wherein at least one amino acid in a CDR is modified to introduce a glycosylated amino acid. In some aspects, the antibody comprises the heavy and light chain CDR sequences of a modified 37E1B5 antibody, wherein the Asn at position 10 of SEQ ID NO:7 is substituted, and the heavy chain CDR2 is modified to introduce a glycosylated amino acid at a different position.

In some aspects, the antibody comprises heavy chain CDR1 and CDR3 sequences from a heavy chain variable region sequence selected from the group consisting of SEQ ID NOs:8, 18, and 19; light chain CDR1, CDR2, and CDR3 sequences from a light chain variable region sequence selected from the group consisting of SEQ ID NO:9, 23 and 24; and heavy chain CDR2 sequence selected from the group consisting of SEQ ID NOs:1-3, wherein the Asn at position 12 (of SEQ ID NOs:1-3) is replaced with Thr or Ser. In some aspects, the heavy chain CDR1 and CDR3 sequences from a heavy chain variable region sequence selected from the group consisting of SEQ ID NOs:10, 20, 21, and 22; light chain CDR1, CDR2, and CDR3 sequences from a light chain variable region sequence selected from the group consisting of SEQ ID NO:11, 25, 26, and 27; and heavy chain CDR2 sequence selected from the group consisting of SEQ ID NOs:4-6, wherein the Asn at position 12 is replaced with Thr or Ser. Again, the CDRs can be determined according to any known method (*e.g.*, Kabat, Chothia, IGMT, or AbM).

In some aspects, the antibody is humanized. In some aspects, the antibody is an Fab, and F(ab)₂, or a single chain Fv (scFv).

Further provided are methods of reducing TGFβ signaling (reducing TGFβ activity, reducing release of mature active TGFβ) in an individual, comprising administering an antibody as described above to the individual, thereby reducing TGFβ signaling in the individual. In some aspects, the individual has at least one condition (disease, disorder) selected from the group consisting of inflammatory bowel disease (IBD), chronic obstructive pulmonary disease (COPD), asthma, arthritis, hepatic fibrosis, a pulmonary fibrotic disorder, an inflammatory brain autoimmune disease, multiple sclerosis, a demyelinating disease, neuroinflammation, kidney disease, adenocarcinoma, squamous carcinoma, glioma, and breast carcinoma; and reducing TGFβ signaling results in amelioration of the condition.

Also provided are methods for producing a recombinant antibody specific for integrin αvβ8 that inhibits release of active, mature TGFβ peptide, comprising recombinantly modifying an antibody (*e.g.*, a monoclonal antibody) that binds β8 to introduce a glycosylated amino acid in a CDR of the antibody, and producing the antibody (*e.g*. by expressing in a cell line or chemically synthesizing), wherein, upon binding to β8, the antibody causes a conformational change that reduces the angle between the head and hybrid domains of β8 compared to β8 not contacted with the antibody. In some aspects, the antibody binds an epitope in the head and/or hybrid domains of β8. In some aspects, the epitope includes the amino acid sequence of SEQ ID NO:16.

In some aspects, the recombinant modification comprises introducing a glycosylation site into the CDR (*e.g*., to allow enzymatic recognition and glycosylation of the site). In some aspects, the recombinant modification comprises introducing an amino acid capable of being glycosylated into the CDR (*e.g*., Asn, Ser, Thr, Tyr). In some aspects, the recombinant modification comprises introducing an unnatural glycosylated amino acid into the CDR. In some aspects, the CDR is the heavy chain CDR2. In some aspects, the glycosylated amino acid corresponds to amino acid position 10 of any one of SEQ ID NOs:1-6 (*e.g*., when the CDR sequence is optimally aligned to any one of SEQ ID NOs:1-6). In some aspects, the antibody reduces the angle between the head and hybrid domains of β8 by at least 5° (*e.g*., at least 6°, 7°, 8°, 9°, 10°, 11°, 12° or more) compared to β8 not contacted with the antibody.

Further provided are methods for producing a glycosylated antibody specific for integrin αvβ8 that inhibits release of active, mature TGFβ peptide, producing an antibody (*e.g*., a monoclonal antibody) that binds β8 and that comprises an amino acid that can be glycosylated in a CDR (*e.g*., Asn, Ser, Thr, Tyr), and chemically glycosylating the amino acid, wherein, upon binding to β8, the antibody causes a conformational change that reduces the angle between the head and hybrid domains of β8 compared to β8 not contacted with the antibody. In some aspects, the producing comprises expressing the antibody in a cell line or chemically synthesizing the antibody. In some aspects, the antibody binds an epitope in the head and/or hybrid domains of β8. In some aspects, the epitope includes the amino acid sequence of SEQ ID NO:16. In some aspects, the CDR is the heavy chain CDR2. In some aspects, the glycosylated amino acid corresponds to amino acid position 10 of any one of SEQ ID NOs:1-6 (*e.g*., when the CDR sequence is optimally aligned to any one of SEQ ID NOs:1-6). In some aspects, the antibody reduces the angle between the head and hybrid domains of β8 by at least 5° (*e.g*., at least 6°, 7°, 8°, 9°, 10°, 11°, 12° or more) compared to β8 not contacted with the antibody.

### BRIEF DESCRIPTION OF THE DRAWINGS

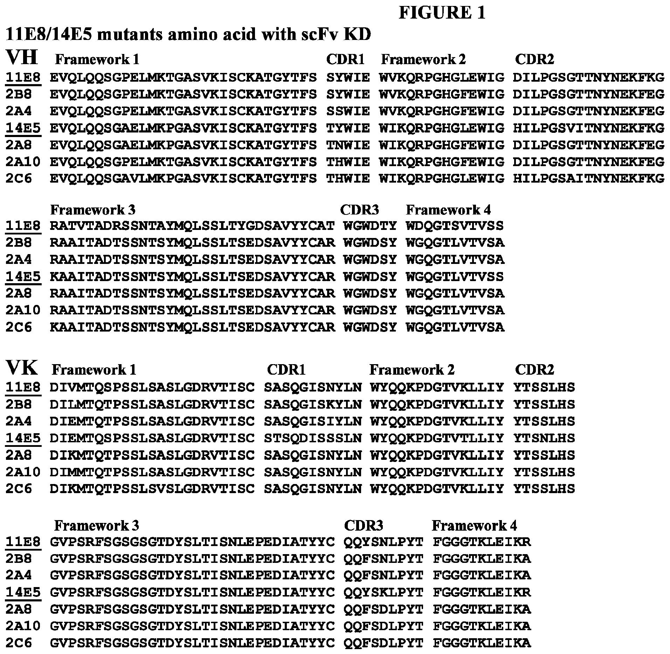

**Figure 1** shows alignment of heavy and light chain variable region sequences of 11E8 and 14E5 antibodies, and variants thereof. Top to bottom, the sequences are designated: SEQ ID NOs:10, 18, 19, 10, 20-22, 9, 23, 24, 11, 25-27. The CDR and framework regions indicated correspond to Kabat numbering.

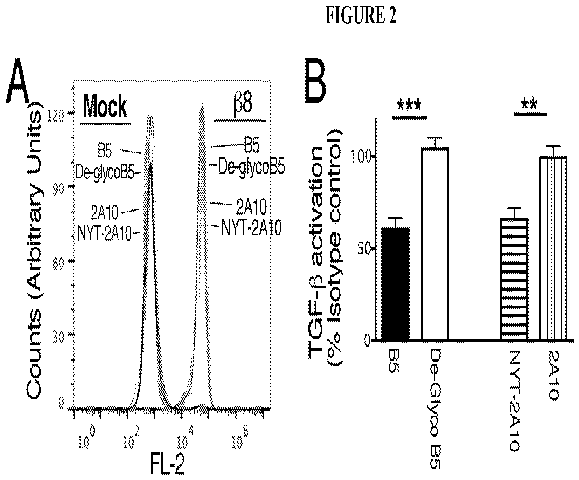

**Figure 2****.** A) Staining of β8 expressing and mock-transfected 293 cells using 37E1B5 (B5) compared to 37E1B5 deglyosylated with PNGaseF (De-glycoB5); and 2A10 compared with 2A10 with the engineered glycan in CDR2 (NYT-2A10). B) TGFβ bioassays of β8 expressing 293 cells treated with 37E1B5, de-glycosylated 37E1B5, glycosylated 2A10 (NYT 2A10), or parental 2A10 (2.5 ug/ml). The results show that glycosylation of heavy chain CDR2 correlates with function blocking ability ***<0.001, **p<0.01. n=4.

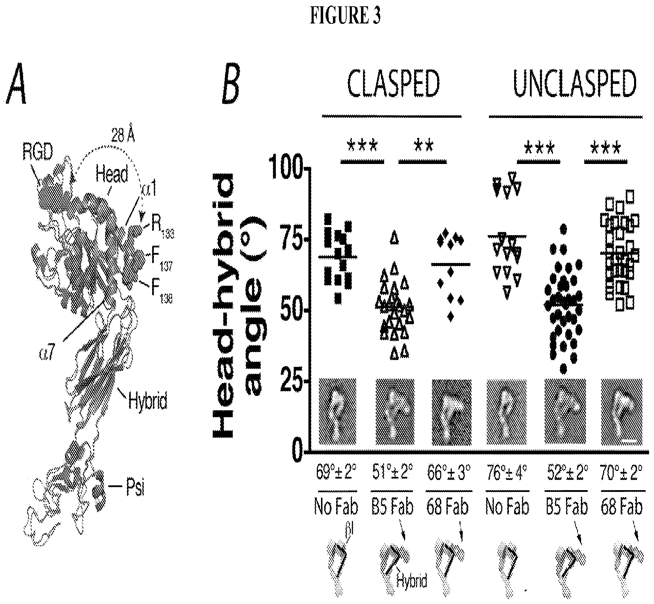

**Figure 3****.** A) Ribbon diagram (PyMOL V1.1r1) of the extended, closed structure of the β8 subunit generated by homology modeling (Modeller) to αvβ3 (PDB 3IJE; Dong et al. (2012) Biochem. 51:8814). Modeled β8 (green) with the α₁ and α₇ helices in red superimposed on αvβ3 (purple). Atoms of B5 epitope on the α1-helix (R₁₃₃, F₁₃₇,F₁₃₈) are indicated. Modeled RGD tripeptide based on PDB 3ZDX (Askari et al. (2010) J Cell Biol 188:891) is shown bound to the ligand-binding pocket in complex with the MIDAS Ca²⁺ cation. Distance from the edge of the ligand-binding pocket (A₁₁₅) to R₁₃₃ of the B5 epitope is 28 Å, indicated by dotted arrows. Head, hybrid and Psi domains are indicated. The αv-subunit and leg domains are not included. B) Image analysis measuring the hybrid-head domain angles of clasped and unclasped αvβ8 with no Fab compared to SEC purified αvβ8-B5 or αvβ8-clone 68 Fabs complexes. Individual angles measured from class averages where the head and hybrid domains were well resolved. *N*=15, 24, 10, 15, 37 and 30 measurements from class-averages of clasped alone (filled squares), clasped + B5 Fab (open upward triangles), clasped + clone 68 Fab (filled diamonds), unclasped alone (open downward triangles), unclasped + B5 Fab (closed circles), unclasped + clone 68 Fab (open squares), respectively. ***P*<0.01, ****P*<0.001 by ANOVA and Tukey's post-test. Insets within the graph below each group are representative EM class averages. The average head-hybrid domain angles (± s.e.m.) are shown below the micrographs. Bar = 10 nm. Cartoons below depict bound Fab with head (βI) and hybrid domains indicated and the angles measured.

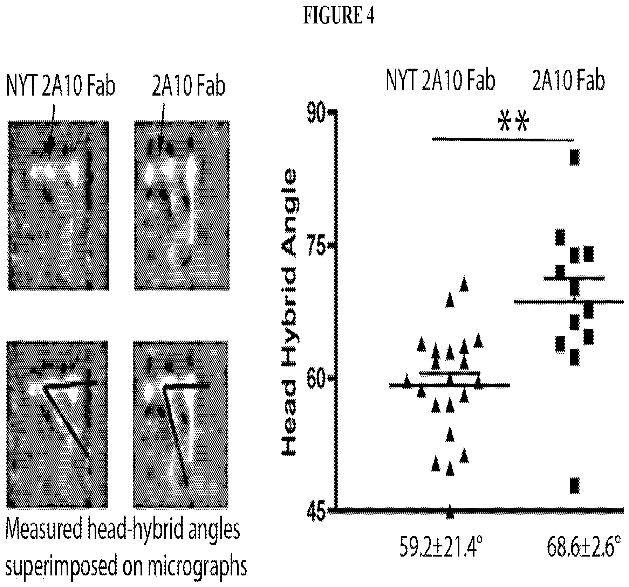

**Figure 4****.** Negative EM staining of αvβ8 in complex with wild-type 2A10 and glycosylated variant NYT-2A10 Fabs, as labeled. Shown are class averages of > 10 individual protein complexes. Angles are superimposed on bottom micrographs to illustrate the head-hybrid angle measurements. The quantification of this data is shown on the right graph with each data point representing a measurement from class-averaged negative EM micrographs.

### DETAILED DESCRIPTION

### I. Introduction

The 37E1B5 antibody is specific for the β8 subunit of integrin αvβ8. Upon binding to its target, 37E1B5 has the unique property of inhibiting release of active mature TGFβ peptide, but not significantly inhibiting adhesion of latent TGFβ to αvβ8 on a αvβ8-expressing cell (*see* WO2011/103490 and WO2013/026004).

The present disclosure reveals that 37E1B5 with a single amino acid substitution removing the glycosylation site in the heavy chain CDR2 retains antigen binding, but loses the ability to block TGFβ activation, indicating that the substituted amino acid is required for TGFβ blocking function, and/or that the glycan at that amino acid is required for TGFβ blocking function. We deglycosylated 37E1B5 chemically, thereby preserving amino acid sequence, and found again that antigen binding is maintained, but TGFβ blocking function is lost. This establishes that the biologic function of 37E1B5 depends on glycosylation in the heavy chain CDR2, and that the functional ability to block TGFβ activation is uncoupled from its ability to bind antigen (β8).

Coinciding structural studies revealed that 37E1B5 Fab with the heavy chain CDR2 glycosylation induces a conformational change in β8. The change is not induced by an Fab prepared from another β8 antibody, clone 68, which binds the same epitope, but does not block TGFβ activation. Glycosylation in the heavy chain CDR2 of other antibodies has been shown, though it typically reduces the desired activity of the antibody, *e.g*., reduces affinity (US Patent No. 5714350).

To test whether the glycan induces a conformational change of αvβ8 that impairs its ability to activate TGFβ, we introduced a glycan into the heavy chain CDR2 of a completely unrelated antibody that binds an epitope on the β8 subunit that overlaps with that of 37E1B5. The 2A10 antibody (variant of 14E5) was selected. The unrelated antibodies have six CDRs with unrelated sequences (*see*, *e.g.,* SEQ ID NOs:21 and 26, compared to SEQ ID NOs:12 and 13). Surprisingly, glycosylation of the heavy chain CDR2 of 2A10 changed its activity so that the antibody could inhibit αvβ8-induced TGFβ activation. Moreover, the glycosylated 2A10 antibody induced a conformational change in β8 similar to that induced by 37E1B1, but not by the non-glycosylated 2A10 antibody.

The present results provide a more global mechanism for modifying antibody activity by introducing glycans in the regions of the antibodies that mediate epitope interaction.

### II. Definitions

Unless defined otherwise, technical and scientific terms used herein have the same meaning as commonly understood by a person of ordinary skill in the art. See, *e.g*., Lackie, DICTIONARY OF CELL AND MOLECULAR BIOLOGY, Elsevier (4th ed. 2007); Sambrook et al., MOLECULAR CLONING, A LABORATORY MANUAL, Cold Springs Harbor Press (Cold Springs Harbor, NY 1989). Any methods, devices and materials similar or equivalent to those described herein can be used in the practice of this present disclosure. The following definitions are provided to facilitate understanding of certain terms used frequently herein and are not meant to limit the scope of the present disclosure.

The terms "anti-αvβ8 antibody," "αvβ8 specific antibody," "αvβ8 antibody," and "anti-αvβ8" are used synonymously herein to refer to an antibody that specifically binds to αvβ8. Similarly, an anti-β8 antibody (and like terms) refer to an antibody that specifically binds to β8. The anti-αvβ8 antibodies and anti-β8 antibodies described herein bind to the protein expressed on αvβ8 expressing cells.

"Nucleic acid" refers to deoxyribonucleotides or ribonucleotides and polymers thereof in either single- or double-stranded form, and complements thereof. The term "polynucleotide" refers to a linear sequence of nucleotides. The term "nucleotide" typically refers to a single unit of a polynucleotide, *i.e.,* a monomer. Nucleotides can be ribonucleotides, deoxyribonucleotides, or modified versions thereof. Examples of polynucleotides contemplated herein include single and double stranded DNA, single and double stranded RNA (including siRNA), and hybrid molecules having mixtures of single and double stranded DNA and RNA.

The words "complementary" or "complementarity" refer to the ability of a nucleic acid in a polynucleotide to form a base pair with another nucleic acid in a second polynucleotide. For example, the sequence A-G-T is complementary to the sequence T-C-A. Complementarity may be partial, in which only some of the nucleic acids match according to base pairing, or complete, where all the nucleic acids match according to base pairing.

A variety of methods of specific DNA and RNA measurements that use nucleic acid hybridization techniques are known to those of skill in the art (*see*, Sambrook, *Id*.). Some methods involve electrophoretic separation (*e.g*., Southern blot for detecting DNA, and Northern blot for detecting RNA), but measurement of DNA and RNA can also be carried out in the absence of electrophoretic separation (*e.g*., quantitative PCR, dot blot, or array).

The words "protein", "peptide", and "polypeptide" are used interchangeably to denote an amino acid polymer or a set of two or more interacting or bound amino acid polymers. The terms apply to amino acid polymers in which one or more amino acid residue is an artificial chemical mimetic of a corresponding naturally occurring amino acid, as well as to naturally occurring amino acid polymers, those containing modified residues, and non-naturally occurring amino acid polymer.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally occurring amino acid, *e.g*., an α carbon that is bound to a hydrogen, a carboxyl group, an amino group, and an R group, *e.*g., homoserine, norleucine, methionine sulfoxide, methionine methyl sulfonium. Such analogs may have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that functions similarly to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical or associated, *e.g.*, naturally contiguous, sequences. Because of the degeneracy of the genetic code, a large number of functionally identical nucleic acids encode most proteins. For instance, the codons GCA, GCC, GCG and GCU all encode the amino acid alanine. Thus, at every position where an alanine is specified by a codon, the codon can be altered to another of the corresponding codons described without altering the encoded polypeptide. Such nucleic acid variations are "silent variations," which are one species of conservatively modified variations. Every nucleic acid sequence herein which encodes a polypeptide also describes silent variations of the nucleic acid. One of skill will recognize that in certain contexts each codon in a nucleic acid (except AUG, which is ordinarily the only codon for methionine, and TGG, which is ordinarily the only codon for tryptophan) can be modified to yield a functionally identical molecule. Accordingly, silent variations of a nucleic acid which encodes a polypeptide is implicit in a described sequence with respect to the expression product, but not with respect to actual probe sequences.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the present disclosure. The following amino acids are typically conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (*see, e.g*., Creighton, Proteins (1984)).

The terms "identical" or "percent identity," in the context of two or more nucleic acids, or two or more polypeptides, refer to two or more sequences or subsequences that are the same or have a specified percentage of nucleotides, or amino acids, that are the same (*i.e.,* about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters, or by manual alignment and visual inspection. *See e.g.,* the NCBI web site at ncbi.nlm.nih.gov/BLAST. Such sequences are then said to be "substantially identical." This definition also refers to, or may be applied to, the compliment of a nucleotide test sequence. The definition also includes sequences that have deletions and/or additions, as well as those that have substitutions. As described below, the algorithms can account for gaps and the like. Typically, identity exists over a region comprising an antibody epitope, or a sequence that is at least about 25 amino acids or nucleotides in length, or over a region that is 50-100 amino acids or nucleotides in length, or over the entire length of the reference sequence.

The term "recombinant" when used with reference, *e.g*., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

The term "heterologous" when used with reference to portions of a nucleic acid indicates that the nucleic acid comprises two or more subsequences that are not found in the same relationship to each other in nature. For instance, the nucleic acid is typically recombinantly produced, having two or more sequences from unrelated genes arranged to make a new functional nucleic acid, e.g., a promoter from one source and a coding region from another source. Similarly, a heterologous protein indicates that the protein comprises two or more subsequences that are not found in the same relationship to each other in nature (*e.g*., a fusion protein).

A "heterologous glycosylated amino acid" refers to an amino acid that is different from the amino acid in the parent antibody (*e.g*., recombinantly introduced) or to an amino acid that is not glycosylated in the parent antibody (*e.g.*, due to the lack of a glycosylation site). In the former case, the amino acid capable of being glycosylated (such as Arg, Ser, Thr, or Cys) or an unnatural amino acid (glycosylated) can be substituted for the amino acid in the parent antibody. In the latter case, the parent antibody can be recombinantly modified to introduce an enzymatically recognized glycosylation site, or the parent antibody can be chemically modified to introduce a glycan.

The term "antibody" refers to a polypeptide comprising a framework region from an immunoglobulin gene, or fragments thereof, that specifically bind and recognize an antigen, *e.g.,* β8, a particular cell surface marker, or any desired target. Typically, the "variable region" contains the antigen-binding region of the antibody (or its functional equivalent) and is most critical in specificity and affinity of binding. See Paul, Fundamental Immunology (2003).

An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

An "isotype" is a class of antibodies defined by the heavy chain constant region. Immunoglobulin genes include the kappa, lambda, alpha, gamma, delta, epsilon, and mu constant region genes. Light chains are classified as either kappa or lambda. Heavy chains are classified as gamma, mu, alpha, delta, or epsilon, which in turn define the isotype classes, IgG, IgM, IgA, IgD and IgE, respectively.

Antibodies can exist as intact immunoglobulins or as any of a number of well-characterized fragments that include specific antigen-binding activity. Such fragments can be produced by digestion with various peptidases. Pepsin digests an antibody below the disulfide linkages in the hinge region to produce F(ab)'₂, a dimer of Fab which itself is a light chain joined to V_{H}-C_{H}1 by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region (*see* Fundamental Immunology (Paul ed., 3d ed. 1993). While various antibody fragments are defined in terms of the digestion of an intact antibody, one of skill will appreciate that such fragments may be synthesized *de novo* either chemically or by using recombinant DNA methodology. Thus, the term antibody, as used herein, also includes antibody fragments either produced by the modification of whole antibodies, or those synthesized *de novo* using recombinant DNA methodologies *(e.g.*, single chain Fv) or those identified using phage display libraries (*see, e.g*., McCafferty et al., Nature 348:552-554 (1990)).

A "monoclonal antibody" refers to a clonal preparation of antibodies with a single binding specificity and affinity for a given epitope on an antigen. A "polyclonal antibody" refers to a preparation of antibodies that are raised against a single antigen, but with different binding specificities and affinities.

As used herein, "V-region" refers to an antibody variable region domain comprising the segments of Framework 1, CDR1, Framework 2, CDR2, Framework 3, CDR3, and Framework 4. These segments are included in the V-segment as a consequence of rearrangement of the heavy chain and light chain V-region genes during B-cell differentiation.

As used herein, "complementarity-determining region (CDR)" refers to the three hypervariable regions in each chain that interrupt the four "framework" regions established by the light and heavy chain variable regions. The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a V_{H} CDR3 is located in the variable domain of the heavy chain of the antibody in which it is found, whereas a V_{L} CDR1 is the CDR1 from the variable domain of the light chain of the antibody in which it is found.

The sequences of the framework regions of different light or heavy chains are relatively conserved within a species. The framework region of an antibody, that is the combined framework regions of the constituent light and heavy chains, serves to position and align the CDRs in three dimensional space.

The amino acid sequences of the CDRs and framework regions can be determined using various well known definitions in the art, *e.g*., Kabat, Chothia, international ImMunoGeneTics database (IMGT), and AbM (*see*, *e.g*., Johnson *et al*., *supra*; Chothia & Lesk, (1987) J. Mol. Biol. 196, 901-917; Chothia et al. (1989) Nature 342, 877-883; Chothia et al. (1992) J. Mol. Biol. 227, 799-817; Al-Lazikani et al., J.Mol.Biol 1997, 273(4)). Definitions of antigen combining sites are also described in the following: Ruiz et al. Nucleic Acids Res., 28, 219-221 (2000); and Lefranc Nucleic Acids Res. Jan 1;29(1):207-9 (2001); MacCallum et al., J. Mol. Biol., 262: 732-745 (1996); and Martin et al, Proc. Natl Acad. Sci. USA, 86, 9268-9272 (1989); Martin, et al, Methods Enzymol., 203: 121-153, (1991); Pedersen et al, Immunomethods, 1, 126, (1992); and Rees et al, In Sternberg M.J.E. (ed.), Protein Structure Prediction. Oxford University Press, Oxford, 141-172 1996).

A "chimeric antibody" is an antibody molecule in which (a) the constant region, or a portion thereof, is altered, replaced or exchanged so that the antigen binding site (variable region, CDR, or portion thereof) is linked to a constant region of a different or altered class, effector function and/or species, or an entirely different molecule which confers new properties to the chimeric antibody (*e.g*., an enzyme, toxin, hormone, growth factor, drug, etc.); or (b) the variable region, or a portion thereof, is altered, replaced or exchanged with a variable region having a different or altered antigen specificity (*e.g*., CDR and framework regions from different species).

The antibody binds to an "epitope" on the antigen. The epitope is the specific antibody binding interaction site on the antigen, and can include a few amino acids or portions of a few amino acids, *e.g.,* 5 or 6, or more, *e.g.,* 20 or more amino acids, or portions of those amino acids. In some cases, the epitope includes non-protein components, *e.g*., from a carbohydrate, nucleic acid, or lipid. In some cases, the epitope is a three-dimensional moiety. Thus, for example, where the target is a protein, the epitope can be comprised of consecutive amino acids, or amino acids from different parts of the protein that are brought into proximity by protein folding (*e.g*., a discontinuous epitope). The same is true for other types of target molecules that form three-dimensional structures.

The term "specifically bind" refers to a molecule (*e.g*., antibody or antibody fragment) that binds to a target with at least 2-fold greater affinity than non-target compounds, *e.g*., at least 4-fold, 5-fold, 6-fold, 7-fold, 8-fold, 9-fold, 10-fold, 20-fold, 25-fold, 50-fold, or 100-fold greater affinity. For example, an antibody that specifically binds β8 will typically bind to β8 with at least a 2-fold greater affinity than a non-β8 target (*e.g*., a different integrin subunit, *e.g.,* β6).

The term "binds" with respect to a cell type (*e*.*g*., an antibody that binds fibrotic cells, hepatocytes, chondrocytes, etc.), typically indicates that an agent binds a majority of the cells in a pure population of those cells. For example, an antibody that binds a given cell type typically binds to at least 2/3 of the cells in a population of the indicated cells (*e.g*., 75, 80, 85, 90, 91, 92, 93, 94, 95, 96, 97, 98, 99, or 100%). One of skill will recognize that some variability will arise depending on the method and/or threshold of determining binding.

As used herein, a first antibody, or an antigen-binding portion thereof, "competes" for binding to a target with a second antibody, or an antigen-binding portion thereof, when binding of the second antibody with the target is detectably decreased in the presence of the first antibody compared to the binding of the second antibody in the absence of the first antibody. The alternative, where the binding of the first antibody to the target is also detectably decreased in the presence of the second antibody, can, but need not be the case. That is, a second antibody can inhibit the binding of a first antibody to the target without that first antibody inhibiting the binding of the second antibody to the target. However, where each antibody detectably inhibits the binding of the other antibody to its cognate epitope or ligand, whether to the same, greater, or lesser extent, the antibodies are said to "cross-compete" with each other for binding of their respective epitope(s). Both competing and cross-competing antibodies are encompassed by the present disclosure. The term "competitor" antibody can be applied to the first or second antibody as can be determined by one of skill in the art. In some cases, the presence of the competitor antibody (*e.g*., the first antibody) reduces binding of the second antibody to the target by at least 10%, *e.g.,* 20%, 30%, 40%, 50%, 60%, 70%, 80%, or more, *e.g*., so that binding of the second antibody to target is undetectable in the presence of the first (competitor) antibody.

The terms "agonist," "activator," "inducer" and like terms refer to molecules that increase activity or expression as compared to a control. Agonists are agents that, e.g., bind to, stimulate, increase, activate, enhance activation, sensitize or upregulate the activity of the target. The expression or activity can be increased 10%, 20%, 30%, 40%, 50%, 60%, 70%, 80%, 90% 100% or more than that in a control. In certain instances, the activation is 1.5-fold, 2-fold, 3-fold, 4-fold, 5-fold, 10-fold, or more in comparison to a control.

The terms "inhibitor," "repressor" or "antagonist" or "downregulator" interchangeably refer to a substance that results in a detectably lower expression or activity level as compared to a control. The inhibited expression or activity can be 10%, 20%, 30%, 40%, 50%, 60%, 70%, 80%, 90% or less than that in a control. In certain instances, the inhibition is 1.5-fold, 2-fold, 3-fold, 4-fold, 5-fold, 10-fold, or more in comparison to a control.

A "control" sample or value refers to a sample that serves as a reference, usually a known reference, for comparison to a test sample. For example, a test sample can be taken from a test condition, *e.g*., in the presence of a test compound, and compared to samples from known conditions, *e.g*., in the absence of the test compound (negative control), or in the presence of a known compound (positive control). A control can also represent an average value gathered from a number of tests or results. One of skill in the art will recognize that controls can be designed for assessment of any number of parameters. For example, a control can be devised to compare therapeutic benefit based on pharmacological data (*e.g*., half-life) or therapeutic measures (*e.g*., comparison of benefit and/or side effects). Controls can be designed for *in vitro* applications. One of skill in the art will understand which controls are valuable in a given situation and be able to analyze data based on comparisons to control values. Controls are also valuable for determining the significance of data. For example, if values for a given parameter are widely variant in controls, variation in test samples will not be considered as significant.

A "label" or a "detectable moiety" is a composition detectable by spectroscopic, photochemical, biochemical, immunochemical, chemical, or other physical means. For example, useful labels include ³²P, fluorescent dyes, electron-dense reagents, enzymes (*e.g*., as commonly used in an ELISA), biotin, digoxigenin, or haptens and proteins or other entities which can be made detectable, *e.g*., by incorporating a radiolabel into a peptide or antibody specifically reactive with a target peptide. Any method known in the art for conjugating an antibody to the label may be employed, *e.g*., using methods described in Hermanson, Bioconjugate Techniques 1996, Academic Press, Inc., San Diego.

A "labeled" molecule (*e.g*., nucleic acid, protein, or antibody) is one that is bound, either covalently, through a linker or a chemical bond, or noncovalently, through ionic, van der Waals, electrostatic, or hydrogen bonds to a label such that the presence of the molecule may be detected by detecting the presence of the label bound to the molecule.

### III. Antibodies specific for αvβ8

Integrin β8 shares a general domain structure with other β integrin subunits, as disclosed in Mould et al. (2006) BMC Cell Biol. 7:24. The head region includes the A-domain, which is followed by the hybrid domain and PSI domain at the "knee" of the protein. The knee is followed by a leg of EGF repeats, followed by a β tail domain, a transmembrane domain, and cytoplasmic domain. Further details are available in the SwissProt entry P26012.

Provided herein are antibodies that specifically bind to integrin αvβ8, but do not significantly bind to other integrins (*e.g*., αvβ6, αvβ3, etc.). The presently disclosed antibodies bind to a specific epitope or epitope region within β8, *e.g*., within the head and/or hybrid domains of β8, such that antibody binding interferes with the ability of αvβ8 to mediate release of active TGFβ. In some aspects, antibody binding causes a conformational change in β8. In some aspects, the antibody is glycosylated in a region that interacts with the β8 epitope, *e.g*., CDR1, CDR2, CDR3, and/or any combination thereof. The epitope can be a conformational (non-linear) or nonconformational epitope. Such an antibody can bind to β8 alone, *i.e.,* the epitope is located within β8, or to a non-linear epitope that comprises parts of both subunits, or an epitope that relies on the interaction of αv and β8.

The present antibodies include the αvβ8 specific antibodies described above, as well as humanized, chimeric, and/or labeled versions thereof, and αvβ8 binding fragments and/or variants thereof. Human isotype IgG1, IgG2, IgG3 or IgG4 can be used for humanized or chimeric antibodies. Some antibodies specifically bind to αvβ8 with a binding affinity greater than or equal to about 10⁷, 10⁸, 10⁹, 10¹⁰, 10¹¹, or 10¹² M⁻¹ (*e.g*., with a Kd in the micromolar (10⁻⁶), nanomolar (10⁻⁹), picomolar (10⁻¹²), or lower range).

In some aspects, the antibody binds to β8 and inhibits TGFβ activation, *e.g*., compared to TGFβ activation in the absence of the antibody. In some aspects, the antibody does not reduce adhesion of cells expressing αvβ8 to TGFβ, that is, the antibody does not reduce αvβ8-mediated cell adhesion to TGFβ. In some aspects, the antibody is glycosylated, and the glycosylated antibody inhibits TGFβ activation, *e.g.*, compared to TGFβ activation in the absence of the antibody, or compared to TGFβ activation in the presence of the non-glycosylated antibody.

In some aspects, the antibody can bind to an epitope on β8 that includes or is within SEQ ID NO:16. The binding site, *i.e.,* epitope, of an antibody raised against a given antigen can be determined using methods known in the art. For example, a competition assay (*e.g*., a competitive ELISA) can be carried out using an antibody with a known epitope. If the test antibody competes for antigen binding, then it likely shares at least part of the same epitope. The epitope can also be localized using domain swapping or selective mutagenesis of the antigen. That is, each region, or each amino acid, of the antigen can be "swapped" out, or substituted with amino acids or components that are known to not interact with the test antibody. If substitution of a given region or amino acid reduces binding of the test antibody to the substituted antigen compared to the non-substituted antigen, then that region or amino acid is likely involved in the epitope.

The Informal Sequence listing provides examples of such antibodies, and shows heavy and light chain variable regions of 37E1B5, 11E8, 14E5 as disclosed in WO2011/103490 and WO2013/026004. CDRs1-3 (Kabat) are indicated by bold underline. Variants of the 11E8 and 14E5 antibodies are shown in Figure 1.

### IV. Detecting TGFβ activity

To determine the effect of an antibody on TGFβ activity, a number of TGFβ bioassays are available. For example, TGFβ activation can be tested in a coculture assay. Test cells expressing αvβ8 are co-cultured with TMLC cells, *i.e.,* mink lung epithelial cells stably transfected with a TGF-β responsive promoter fragment driving the luciferase gene (Abe et al. (1994) Annal Biochem 216:276). TMLC cells are highly responsive to TGFβ with a very low background of TGFβ activation. TMLC cells can thus be used in coculture with other cell lines or cell-free fractions to test for the presence of active TGFβ using luminescence as a readout. Assays can be performed in the presence or absence of a control TGFβ-blocking antibody (*e.g*., 10 µg/ml, 1D11; R&D Systems).

To measure active TGFβ in tumor tissue, equal weights of tumor tissue can be minced and incubated in sterile DME for 30 min at 4°C. The supernatants containing active TGFβ can be harvested after centrifugation (20 g) at 4°C. The pellets can then be incubated in serum-free DME for 20 min at 80°C to activate SLC, after which the supernatants can be harvested. The supernatants containing active or heat-activated (latent) TGFβ are then added to pre-plated TMLC cells with or without 1D11. For protease inhibitor assays, inhibitors are added at the initiation of the coculture. The maximal dose of each inhibitor is defined as the highest concentration that does not inhibit the ability of the TMLC cells to respond to recombinant active TGFβ. To measure soluble TGFβ activity from cultured cells, cells are incubated in 100 µl of complete medium with or without 37E1 or 10D5 for 1 h at 37°C with gentle rotation. Cell-free supernatants are harvested by centrifugation (20 g) for 5 min at 4°C and then added to preplated TMLC cells in the presence or absence of 1D11. For soluble receptor assays, conditioned medium obtained from overnight cultures of cells is used. Relative luciferase units are defined as activity minus the background activity of the TMLC reporter cells.

### V. Methods for producing and modifying antibodies

For preparation and use of suitable antibodies as described herein, *e.g*., recombinant or monoclonal antibodies, many techniques known in the art can be used (*see, e.g.*, Kohler & Milstein, Nature 256:495-497 (1975); Kozbor et al., Immunology Today 4: 72 (1983); Cole et al., pp. 77-96 in Monoclonal Antibodies and Cancer Therapy, Alan R. Liss, Inc. (1985); Coligan, Current Protocols in Immunology (1991); Harlow & Lane, Antibodies, A Laboratory Manual (1988); and Goding, Monoclonal Antibodies: Principles and Practice (2d ed. 1986)). The genes encoding the heavy and light chains of an antibody of interest can be cloned from a cell, *e.g*., the genes encoding a monoclonal antibody can be cloned from a hybridoma and used to produce a recombinant monoclonal antibody. Gene libraries encoding heavy and light chains of monoclonal antibodies can also be made from hybridoma or plasma cells. Random combinations of the heavy and light chain gene products generate a large pool of antibodies with different antigenic specificity (*see*, *e.g*., Kuby, Immunology (3rd ed. 1997)). Techniques for the production of single chain antibodies or recombinant antibodies (U.S. Patent 4,946,778, U.S. Patent No. 4,816,567) can be adapted to produce antibodies to polypeptides of this present disclosure. Also, transgenic mice, or other organisms such as other mammals, can be used to express humanized or human antibodies (*see*, *e.g*., U.S. Patent Nos. 5,545,807; 5,545,806; 5,569,825; 5,625,126; 5,633,425; 5,661,016, Marks et al., Bio/Technology 10:779-783 (1992); Lonberg et al., Nature 368:856-859 (1994); Morrison, Nature 368:812-13 (1994); Fishwild et al., Nature Biotechnology 14:845-51 (1996); Neuberger, Nature Biotechnology 14:826 (1996); and Lonberg & Huszar, Intern. Rev. Immunol. 13:65-93 (1995)). Alternatively, phage or yeast display technology can be used to identify antibodies and heteromeric Fab fragments that specifically bind to selected antigens (*see*, *e.g*., McCafferty et al., Nature 348:552-554 (1990); Marks et al., Biotechnology 10:779-783 (1992); Lou et al. (2010) PEDS 23:311). Antibodies can also be made bispecific, i.e., able to recognize two different antigens (*see*, *e.g*., WO 93/08829, Traunecker et al., EMBO J. 10:3655-3659 (1991); and Suresh et al., Methods in Enzymology 121:210 (1986)). Antibodies can also be heteroconjugates, *e.g*., two covalently joined antibodies, or immunotoxins (*see*, *e.g*., U.S. Patent No. 4,676,980 , WO 91/00360; WO 92/200373; and EP 03089).

In the context of the present disclosure, recombinant antibodies can be produced from a parent monoclonal (or polyclonal) antibody such that an amino acid that can be glycosylated (*e.g*., chemically or enzymatically) or a glycosylation site (recognized by a glycosylating enzyme) is incorporated into the variable region, *e.g*., a CDR. The glycosylation can be N-linked, O-linked, phospho-linked, or C-linked. N-linked glycosylation typically occurs on Asn. A canonical N-linkage glycosylation site is Asn-Xaa-Ser/Thr/Cys, where Xaa is not Pro. O-linked glycosylation occurs on Ser, Thr, and Tyr residues, as well as hydroxylysine and hydroxyproline, and can be carried out by *O*-GlcNAc transferase. Phospho-linkage can occur on phosphoserine. C-linkage can occur on Trp, and a canonical C-linkage site is Trp-Ser/Thr-Xaa-Cys.

The antibody can be recombinantly produced such that a glycosylated amino acid is introduced during translation of the antibody, instead of during post-translational processing or *in vitro* manipulation. Systems for introducing unnatural amino acids are disclosed, *e.g.,* by Chin (2011) EMBO 30:2307; Kaya et al. (2009) ChemBioChem 10:2858; Wang et al. (2006) Annu Rev Biophys Biomol Rev 35:225.

Antibodies can be produced using any number of expression systems, including prokaryotic and eukaryotic expression systems. In some aspects, the expression system is a mammalian cell expression, such as a hybridoma, or a CHO cell expression system. Many such systems are widely available from commercial suppliers. In aspects in which an antibody comprises both a V_{H} and V_{L} region, the V_{H} and V_{L} regions may be expressed using a single vector, *e.g*., in a di-cistronic expression unit, or under the control of different promoters. In other aspects, the V_{H} and V_{L} region may be expressed using separate vectors. A V_{H} or V_{L} region as described herein may optionally comprise a methionine at the N-terminus.

An antibody as described herein can also be produced in various formats, including as a Fab, a Fab', a F(ab')₂, a scFv, or a dAB. The antibody fragments can be obtained by a variety of methods, including, digestion of an intact antibody with an enzyme, such as pepsin (to generate (Fab')₂ fragments) or papain (to generate Fab fragments); or *de novo* synthesis. Antibody fragments can also be synthesized using recombinant DNA methodology. In some aspects, an anti-β8 antibody comprises F(ab')₂ fragments that specifically bind β8. An antibody of the present disclosure can also include a human constant region. See, *e.g*., Fundamental Immunology (Paul ed., 4d ed. 1999); Bird, et al., Science 242:423 (1988); and Huston, et al., Proc. Natl. Acad. Sci. USA 85:5879 (1988).

Methods for humanizing or primatizing non-human antibodies are also known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers (*see*, *e.g*., Jones et al., Nature 321:522-525 (1986); Riechmann et al., Nature 332:323-327 (1988); Verhoeyen et al., Science 239:1534-1536 (1988) and Presta, Curr. Op. Struct. Biol. 2:593-596 (1992)), by substituting rodent CDRs or CDR sequences for the corresponding sequences of a human antibody. Such humanized antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

In some cases, the antibody or antibody fragment can be conjugated to another molecule, *e.g*., polyethylene glycol (PEGylation) or serum albumin, to provide an extended half-life *in vivo.* Examples of PEGylation of antibody fragments are provided in Knight et al. Platelets 15:409, 2004 (for abciximab); Pedley et al., Br. J. Cancer 70:1126, 1994 (for an anti-CEA antibody); Chapman et al., Nature Biotech. 17:780, 1999; and Humphreys, et al., Protein Eng. Des. 20: 227,2007). The antibody or antibody fragment can also be labeled, or conjugated to a therapeutic agent as described below.

The specificity of antibody binding can be defined in terms of the comparative dissociation constants (Kd) of the antibody for the target (*e.g*., β8) as compared to the dissociation constant with respect to the antibody and other materials in the environment or unrelated molecules in general. Typically, the Kd for the antibody with respect to the unrelated material will be at least 2-fold, 3-fold, 4-fold, 5-fold, 10-fold, 20-fold, 50-fold, 100-fold, 200-fold or higher than Kd with respect to the target.

The desired affinity for an antibody, *e.g*., high (pM to low nM), medium (low nM to 100nM), or low (about 100nM or higher), may differ depending upon whether it is being used as a diagnostic or therapeutic. For example, an antibody with medium affinity may be more successful in localizing to desired tissue as compared to one with a high affinity. Thus, antibodies having different affinities can be used for diagnostic and therapeutic applications.

A targeting moiety will typically bind with a Kd of less than about 1000 nM, *e.g*., less than 250, 100, 50, 20 or lower nM. In some aspects, the Kd of the affinity agent is less than 15, 10, 5, or 1 nM. In some aspects, the Kd is 1-100 nM, 0.1-50 nM, 0.1-10 nM, or 1-20 nM. The value of the dissociation constant (Kd) can be determined by well-known methods, and can be computed even for complex mixtures by methods as disclosed, *e.g.,* in Caceci et al., Byte (1984) 9:340-362.

Affinity of an antibody, or any targeting agent, for a target can be determined according to methods known in the art, *e.g.,* as reviewed in Ernst et al. Determination of Equilibrium Dissociation Constants, Therapeutic Monoclonal Antibodies (Wiley & Sons ed. 2009).

Quantitative ELISA, and similar array-based affinity methods can be used. ELISA (Enzyme linked immunosorbent signaling assay) is an antibody-based method. In some cases, an antibody specific for target of interest is affixed to a substrate, and contacted with a sample suspected of containing the target. The surface is then washed to remove unbound substances. Target binding can be detected in a variety of ways, *e.g*., using a second step with a labeled antibody, direct labeling of the target, or labeling of the primary antibody with a label that is detectable upon antigen binding. In some cases, the antigen is affixed to the substrate (*e.g*., using a substrate with high affinity for proteins, or a Strepavidin-biotin interaction) and detected using a labeled antibody (or other targeting moiety). Several permutations of the original ELISA methods have been developed and are known in the art (see Lequin (2005) Clin. Chem. 51:2415-18 for a review).

The Kd, Kon, and Koff can also be determined using surface plasmon resonance (SPR), *e.g.,* as measured by using a Biacore T100 system. SPR techniques are reviewed, *e.g.*, in Hahnfeld et al. Determination of Kinetic Data Using SPR Biosensors, Molecular Diagnosis of Infectious Diseases (2004). In a typical SPR experiment, one interactant (target or targeting agent) is immobilized on an SPR-active, gold-coated glass slide in a flow cell, and a sample containing the other interactant is introduced to flow across the surface. When light of a given frequency is shined on the surface, the changes to the optical reflectivity of the gold indicate binding, and the kinetics of binding.

Binding affinity can also be determined by anchoring a biotinylated interactant to a streptaviden (SA) sensor chip. The other interactant is then contacted with the chip and detected, *e.g.,* as described in Abdessamad et al. (2002) Nuc. Acids Res. 30:e45.

### VI. Examples

### Example 1

A glycan in CDR2 of 37E1B5 is required for full TGFβ blocking activity, but not binding to β8. We tested a 37E1B5 variant with a single amino acid substitution to disrupt the glycosylation site in heavy chain CDR2 (amino acid position in SEQ ID NO:7). The antibody was not able to block TGFβ activation and release.

The presence of a canonical glycosylation site, however, does not necessarily indicate that glycosylation occurs at that site. The change in the activity of the 37E1B5 variant could thus be due to either lack of glycosylation, or to the substituted amino acid.

We deglycosylated 37E1B5 using Peptide -N-Glycosidase F (PNGase F), thereby preserving the amino acid sequence (heavy chain CDR2 is represented by SEQ ID NO:7). As shown in Figure 2A, 37E1B5 (B5) and deglycosylated 37E1B5 (de-glycoB5) bind to β8 with equal affinity. Figure 2B, however, shows that the deglycosylated 37E1B5 does not block TGFβ activation. The results indicate that glycosylation in the heavy chain CDR2 of 37E1B5 affects the ability of the antibody to block TGFβ activation.

We engineered a glycosylation site into the heavy chain of the CDR2 of an unrelated antibody, 2A10 (*see* Figure 1). The 2A10 antibody is a variant of the 14E5 monoclonal antibody, and binds an epitope that overlaps with that of 37E1B5, but does not inhibit TGFβ activation by αvβ8. The heavy and light chain variable region sequences of the 2A10 and 37E1B5 antibodies share 38% and 44% identity, respectively.

A glycosylation site was introduced into the 2A10 CDR2 heavy chain by substituting the Asn at position 12 of SEQ ID NO:2 with a Thr. This results in glycosylation at the Asn at position 10 of SEQ ID NO:2.

Surprisingly, we observed results similar to those of glycosylated and non-glycosylated forms of 37E1B5 with glycosylated and non-glycosylated forms of 2A10. That is, glycosylation of 2A10 converted the function of the 2A10 antibody so that it could inhibit TGFβ activation by αvβ8. Figure 2A shows that non-glycosylated 2A10 (2A10) and glycosylated 2A10 (NYT-2A10) bind to β8 with equal affinity. Figure 2B, however, shows that the non-glycosylated 2A10 does not block TGFβ activation, while glycosylated 2A10 does. The results indicate that glycosylation has a more global role in the ability of a β8-binding antibody to block TGFβ activation.

### Example 2

We carried out structural studies with the Fab of 37E1B5 and the Fab of the antibody clone 68. Clone 68 binds an epitope on β8 that overlaps with that of 37E1B5, but it does not inhibit TGFβ activation and release.

A ribbon diagram of the head and hybrid domains of β8 is shown in Figure 3A. The figure indicates the normal orientation of β8 bound to RGD (upper left), and the amino acids included in the epitope of 37E1B5 (right).

Figure 3B shows that the TGFβ-blocking Fab 37E1B5 causes a subtle inward bending of the β8 head-hybrid domain angle upon binding. This change is not observed for the no-antibody control, or for the non-blocking antibody 68 Fab. The 37E1B5-dependent bending occurred in both the clasped and unclasped forms of β8. The results indicate that the TGFβ inhibiting activity of 37E1B5 is mediated by the conformational change in β8 upon binding to the antibody.

### Example 3

The combined results thus far demonstrate that the ability of a β8-specific antibody to inhibit αvβ8-mediated activation of TGFβ is affected by glycosylation of the heavy chain CDR2, and by the ability of the antibody to induce a conformational change in β8 upon binding.

To determine if glycosylation is involved in the conformational change in β8, we carried out structural analysis of the glycosylated and non-glycosylated 2A10 antibody Fabs. As with 37E1B5, a glycan in heavy chain CDR2 of 2A10 induces a conformational change in the head-hybrid angle of β8. Figure 4 shows the results. Glycosylated 2A10 (NYT 2A10 Fab) induces a similar reduction in the head-hybrid angle of β8 as glycosylated 37E1B5. The results indicate that glycosylation of an antibody that binds the head and/or hybrid region of β8 can induce a conformational change in β8, and inhibit its ability to activate TGFβ.

### VII. Informal sequence listing

Heavy chain CDR2 of 11E8 antibody (SEQ ID NO:1)
   Asp Ile Leu Pro Gly Ser Gly Thr Thr Asn Tyr Asn Glu Lys Phe Lys
Heavy chain CDR2 of 11E8mut94 and 14E5 (2A10) antibodies (SEQ ID NO:2)
   Asp Ile Leu Pro Gly Ser Gly Thr Thr Asn Tyr Asn Glu Lys Phe Glu
Heavy chain CDR2 of 11E8mut39 (SEQ ID NO:3)
   His Thr Leu Pro Gly Ser Gly Thr Thr Asn Tyr Asn Glu Lys Phe Lys
Heavy chain CDR2 of 14E5 antibody (SEQ ID NO:4)
   His Ile Leu Pro Gly Ser Val Ile Thr Asn Tyr Asn Glu Lys Phe Lys
Heavy chain CDR2 of 14E5mut68 antibody (SEQ ID NO:5)
   Asp Ile Leu Pro Gly Ser Gly Thr Thr Asn Tyr Asn Glu Lys Phe Lys
Heavy chain CDR2 of 14E5 (2C6) antibody (SEQ ID NO:6)
   His Ile Leu Pro Gly Ser Ala Ile Thr Asn Tyr Asn Glu Lys Phe Lys
Heavy chain CDR2 of 37E1B5 and humanized 37E1B5 antibodies (SEQ ID NO:7)
   Glu Ile Asn Pro Asp Ser Ser Thr Ile Asn Tyr Thr Ser Ser Leu Lys
Heavy chain variable region of 11E8 antibody (SEQ ID NO:8) 
Glu Val Gln Leu Gln Gln Ser Gly Pro Glu Leu Met Lys Thr Gly Ala
Ser Val Lys Ile Ser Cys Lys Ala Thr Gly Tyr Thr Phe Ser Ser Tyr
Trp Ile Glu Trp Val Lys Gln Arg Pro Gly His Gly Leu Glu Trp Ile
Gly Asp Ile Leu Pro Gly Ser Gly Thr Thr Asn Tyr Asn Glu Lys Phe
Lys Gly Arg Ala Thr Val Thr Ala Asp Arg Ser Ser Asn Thr Ala Tyr
Met Gln Leu Ser Ser Leu Thr Tyr Gly Asp Ser Ala Val Tyr Tyr Cys
Ala Thr Trp Gly Trp Asp Thr Tyr Trp Asp Gln Gly Thr Ser Val Thr
Val Ser Ser
Light chain variable region of 11E8 antibody (SEQ ID NO:9) 
Asp Ile Val Met Thr Gln Ser Pro Ser Ser Leu Ser Ala Ser Leu Gly
Asp Arg Val Thr Ile Ser Cys Ser Ala Ser Gln Gly Ile Ser Asn Tyr
Leu Asn Trp Tyr Gln Gln Lys Pro Asp Gly Thr Val Lys Leu Leu Ile
Tyr Tyr Thr Ser Ser Leu His Ser Gly Val Pro Ser Arg Phe Ser Gly
Ser Gly Ser Gly Thr Asp Tyr Ser Leu Thr Ile Ser Asn Leu Glu Pro
Glu Asp Ile Ala Thr Tyr Tyr Cys Gln Gln Tyr Ser Asn Leu Pro Tyr
Thr Phe Gly Gly Gly Thr Lys Leu Glu Ile Lys Arg
Heavy chain variable region of 14E5 antibody (SEQ ID NO:10) 
Glu Val Gln Leu Gln Gln Ser Gly Ala Glu Leu Met Lys Pro Gly Ala
Ser Val Lys Ile Ser Cys Lys Ala Thr Gly Tyr Thr Phe Ser Thr Tyr
Trp Ile Glu Trp Ile Lys Gln Arg Pro Gly His Gly Leu Glu Trp Ile
Gly His Ile Leu Pro Gly Ser Val Ile Thr Asn Tyr Asn Glu Lys Phe
Lys Gly Lys Ala Ala Ile Thr Ala Asp Thr Ser Ser Asn Thr Ser Tyr
Met Gln Leu Ser Ser Leu Thr Ser Glu Asp Ser Ala Val Tyr Tyr Cys
Ala Arg Trp Gly Trp Asp Ser Tyr Trp Gly Gln Gly Thr Leu Val Thr
Val Ser Ser
Light chain variable region of 14E5 antibody (SEQ ID NO:11) 
Asp Ile Glu Met Thr Gln Ser Pro Ser Ser Leu Ser Ala Ser Leu Gly
Asp Arg Val Thr Ile Ser Cys Ser Thr Ser Gln Asp Ile Ser Ser Ser
Leu Asn Trp Tyr Gln Gln Lys Pro Asp Gly Thr Val Thr Leu Leu Ile
Tyr Tyr Thr Ser Asn Leu His Ser Gly Val Pro Ser Arg Phe Ser Gly
Ser Gly Ser Gly Thr Asp Tyr Ser Leu Thr Ile Ser Asn Leu Glu Pro
Glu Asp Ile Ala Thr Tyr Tyr Cys Gln Gln Tyr Ser Lys Leu Pro Tyr
Thr Phe Gly Gly Gly Thr Lys Leu Glu Ile Lys Arg
Heavy chain variable region of 37E1B5 antibody (SEQ ID NO:12) 
Glu Val Gln Leu Val Glu Ser Gly Gly Gly Leu Val Gln Pro Gly Gly
Ser Leu Asn Leu Ser Cys Ala Val Ser Gly Phe Val Phe Ser Arg Tyr
Trp Met Ser Trp Val Arg Gln Ala Pro Gly Lys Gly Leu Glu Trp Ile
Gly Glu Ile Asn Pro Asp Ser Ser Thr Ile Asn Tyr Thr Ser Ser Leu
Lys Asp Lys Phe Ile Ile Ser Arg Asp Asn Ala Lys Asn Thr Leu Tyr
Leu Gln Met Asn Lys Val Arg Ser Glu Asp Thr Ala Leu Tyr Tyr Cys
Ala Cys Leu Ile Thr Thr Glu Asp Tyr Trp Gly Gln Gly Thr Ser Val
Thr Val Ser Ser
Light chain variable region of 37E1B5 antibody (SEQ ID NO:13) 
Gln Ile Val Leu Thr Gln Ser Pro Ser Ser Met Tyr Ala Ser Leu Gly
Glu Arg Val Thr Ile Pro Cys Lys Ala Ser Gln Asp Ile Asn Ser Tyr
Leu Ser Trp Phe Gln Gln Lys Pro Gly Lys Ser Pro Lys Thr Leu Ile
Tyr Tyr Ala Asn Arg Leu Val Asp Gly Val Pro Ser Arg Phe Ser Gly
Ser Gly Ser Gly Gln Asp Tyr Ser Leu Thr Ile Ser Ser Leu Glu Tyr
Glu Asp Met Gly Ile Tyr Tyr Cys Leu Gln Tyr Asp Glu Phe Pro Tyr
Thr Phe Gly Gly Gly Thr Lys Leu Glu Ile Lys Ala
Heavy chain variable region of humanized 37E1B5 antibody (SEQ ID NO:14) 
Glu Val Gln Leu Val Glu Ser Gly Gly Gly Leu Val Gln Pro Gly Gly
Ser Leu Arg Leu Ser Cys Ala Val Ser Gly Phe Val Phe Ser Arg Tyr
Trp Met Ser Trp Val Arg Gln Ala Pro Gly Lys Gly Leu Glu Trp Ile
Gly Glu Ile Asn Pro Asp Ser Ser Thr Ile Asn Tyr Thr Ser Ser Leu
Lys Asp Arg Phe Thr Ile Ser Arg Asp Asn Ala Lys Asn Ser Leu Tyr
Leu Gln Met Asn Ser Leu Arg Ala Glu Asp Thr Ala Val Tyr Tyr Cys
Ala Ser Leu Ile Thr Thr Glu Asp Tyr Trp Gly Gln Gly Thr Thr Val
Thr Val Ser Ser
Light chain variable region of humanized 37E1B5 antibody (SEQ ID NO:15) 
Glu Ile Val Leu Thr Gln Ser Pro Ser Ser Leu Ser Leu Ser Pro Gly
Glu Arg Val Thr Ile Thr Cys Lys Ala Ser Gln Asp Ile Asn Ser Tyr
Leu Ser Trp Tyr Gln Gln Lys Pro Gly Lys Ala Pro Lys Leu Leu Ile
Tyr Tyr Ala Asn Arg Leu Val Asp Gly Val Pro Ala Arg Phe Ser Gly
Ser Gly Ser Gly Gln Asp Tyr Thr Leu Thr Ile Ser Ser Leu Glu Pro
Glu Asp Phe Ala Val Tyr Tyr Cys Leu Gln Tyr Asp Glu Phe Pro Tyr
Thr Phe Gly Gly Gly Thr Lys Leu Glu Ile Lys Arg
Exemplary integrin beta 8 epitope (SEQ ID NO:16)
   Ser Arg Lys Met Ala Phe Phe
Full length human integrin beta 8 (SEQ ID NO:17)

Met Cys Gly Ser Ala Leu Ala Phe Phe Thr Ala Ala Phe Val Cys Leu
Gln Asn Asp Arg Arg Gly Pro Ala Ser Phe Leu Trp Ala Ala Trp Val
Phe Ser Leu Val Leu Gly Leu Gly Gln Gly Glu Asp Asn Arg Cys Ala
Ser Ser Asn Ala Ala Ser Cys Ala Arg Cys Leu Ala Leu Gly Pro Glu
Cys Gly Trp Cys Val Gln Glu Asp Phe Ile Ser Gly Gly Ser Arg Ser
Glu Arg Cys Asp Ile Val Ser Asn Leu Ile Ser Lys Gly Cys Ser Val
Asp Ser Ile Glu Tyr Pro Ser Val His Val Ile Ile Pro Thr Glu Asn
Glu Ile Asn Thr Gln Val Thr Pro Gly Glu Val Ser Ile Gln Leu Arg
Pro Gly Ala Glu Ala Asn Phe Met Leu Lys Val His Pro Leu Lys Lys
Tyr Pro Val Asp Leu Tyr Tyr Leu Val Asp Val Ser Ala Ser Met His
Asn Asn Ile Glu Lys Leu Asn Ser Val Gly Asn Asp Leu Ser Arg Lys
Met Ala Phe Phe Ser Arg Asp Phe Arg Leu Gly Phe Gly Ser Tyr Val
Asp Lys Thr Val Ser Pro Tyr Ile Ser Ile His Pro Glu Arg Ile His
Asn Gln Cys Ser Asp Tyr Asn Leu Asp Cys Met Pro Pro His Gly Tyr
Ile His Val Leu Ser Leu Thr Glu Asn Ile Thr Glu Phe Glu Lys Ala
Val His Arg Gln Lys Ile Ser Gly Asn Ile Asp Thr Pro Glu Gly Gly
Phe Asp Ala Met Leu Gln Ala Ala Val Cys Glu Ser His Ile Gly Trp
Arg Lys Glu Ala Lys Arg Leu Leu Leu Val Met Thr Asp Gln Thr Ser
His Leu Ala Leu Asp Ser Lys Leu Ala Gly Ile Val Val Pro Asn Asp
Gly Asn Cys His Leu Lys Asn Asn Val Tyr Val Lys Ser Thr Thr Met
Glu His Pro Ser Leu Gly Gln Leu Ser Glu Lys Leu Ile Asp Asn Asn
Ile Asn Val Ile Phe Ala Val Gln Gly Lys Gln Phe His Trp Tyr Lys
Asp Leu Leu Pro Leu Leu Pro Gly Thr Ile Ala Gly Glu Ile Glu Ser
Lys Ala Ala Asn Leu Asn Asn Leu Val Val Glu Ala Tyr Gln Lys Leu
Ile Ser Glu Val Lys Val Gln Val Glu Asn Gln Val Gln Gly Ile Tyr
Phe Asn Ile Thr Ala Ile Cys Pro Asp Gly Ser Arg Lys Pro Gly Met
Glu Gly Cys Arg Asn Val Thr Ser Asn Asp Glu Val Leu Phe Asn Val
Thr Val Thr Met Lys Lys Cys Asp Val Thr Gly Gly Lys Asn Tyr Ala
Ile Ile Lys Pro Ile Gly Phe Asn Glu Thr Ala Lys Ile His Ile His
Arg Asn Cys Ser Cys Gln Cys Glu Asp Asn Arg Gly Pro Lys Gly Lys
Cys Val Asp Glu Thr Phe Leu Asp Ser Lys Cys Phe Gln Cys Asp Glu
Asn Lys Cys His Phe Asp Glu Asp Gln Phe Ser Ser Glu Ser Cys Lys
Ser His Lys Asp Gln Pro Val Cys Ser Gly Arg Gly Val Cys Val Cys
Gly Lys Cys Ser Cys His Lys Ile Lys Leu Gly Lys Val Tyr Gly Lys
Tyr Cys Glu Lys Asp Asp Phe Ser Cys Pro Tyr His His Gly Asn Leu
Cys Ala Gly His Gly Glu Cys Glu Ala Gly Arg Cys Gln Cys Phe Ser
Gly Trp Glu Gly Asp Arg Cys Gln Cys Pro Ser Ala Ala Ala Gln His
Cys Val Asn Ser Lys Gly Gln Val Cys Ser Gly Arg Gly Thr Cys Val
Cys Gly Arg Cys Glu Cys Thr Asp Pro Arg Ser Ile Gly Arg Phe Cys
Glu His Cys Pro Thr Cys Tyr Thr Ala Cys Lys Glu Asn Trp Asn Cys
Met Gln Cys Leu His Pro His Asn Leu Ser Gln Ala Ile Leu Asp Gln
Cys Lys Thr Ser Cys Ala Leu Met Glu Gln Gln His Tyr Val Asp Gln
Thr Ser Glu Cys Phe Ser Ser Pro Ser Tyr Leu Arg Ile Phe Phe Ile
Ile Phe Ile Val Thr Phe Leu Ile Gly Leu Leu Lys Val Leu Ile Ile
Arg Gln Val Ile Leu Gln Trp Asn Ser Asn Lys Ile Lys Ser Ser Ser
Asp Tyr Arg Val Ser Ala Ser Lys Lys Asp Lys Leu Ile Leu Gln Ser
Val Cys Thr Arg Ala Val Thr Tyr Arg Arg Glu Lys Pro Glu Glu Ile
Lys Met Asp Ile Ser Lys Leu Asn Ala His Glu Thr Phe Arg Cys Asn
Phe

## Claims

1. A method of producing a recombinant antibody that inhibits release of active, mature TGFβ peptide, comprising:
identifying an antibody that binds an epitope in the head and/or hybrid domains of β8;
recombinantly modifying the heavy chain CDR2 sequence to introduce a glycosylation site; and
expressing the antibody, wherein, upon binding to β8, the antibody causes a conformational change that reduces the angle between the head and hybrid domains of β8 compared to β8 not contacted with the antibody.

2. A method of producing a glycosylated antibody that inhibits release of active, mature TGFβ peptide, comprising:
identifying an antibody that binds an epitope in the head and/or hybrid domains of β8, and that comprises an amino acid that can be glycosylated in the heavy chain CDR2 sequence;
expressing the antibody; and
chemically glycosylating the amino acid, wherein, upon binding to β8, the antibody causes a conformational change that reduces the angle between the head and hybrid domains of β8 compared to β8 contacted with the antibody without chemical glycosylation.

3. The method of claim 1 or 2, wherein the recombinant or glycosylated antibody binds an epitope comprising SEQ ID NO: 16.

4. The method of any one of claims 1-3, wherein, upon binding β8, the recombinant or glycosylated antibody reduces the angle between the head and hybrid domains of β8 by at least 5° compared to β8 not contacted with the antibody.

5. The method of any one of claims 1-4, wherein the amino acid that is glycosylated corresponds to amino acid position 10 in any one of SEQ ID NOs: 1-6.

## Patentansprüche

1. Verfahren zur Erzeugung eines rekombinanten Antikörpers, der die Freisetzung von aktivem, reifem TGFβ-Peptid hemmt, umfassend:
Identifizieren eines Antikörpers, der ein Epitop in den Kopf- und/oder Hybriddomänen von β8 bindet;
rekombinantes Modifizieren der Schwere-Kette-CDR2-Sequenz zur Einführung einer Glykosylierungsstelle;
und
Exprimieren des Antikörpers, wobei der Antikörper nach Bindung an β8 eine Konformationsänderung hervorruft, durch die der Winkel zwischen den Kopf- und Hybriddomänen von β8 verglichen mit nicht mit dem Antikörper in Kontakt gebrachtem β8 verkleinert wird.

2. Verfahren zur Erzeugung eines glykosylierten Antikörpers, der die Freisetzung von aktivem, reifem TGFβ-Peptid hemmt, umfassend:
Identifizieren eines Antikörpers, der ein Epitop in den Kopf- und/oder Hybriddomänen von β8 bindet und der eine Aminosäure, die glykosyliert werden kann, in der Schwere-Kette-CDR2-Sequenz umfasst;
Exprimieren des Antikörpers; und
chemisches Glykosylieren der Aminosäure, wobei der Antikörper nach Bindung an β8 eine Konformationsänderung hervorruft, durch die der Winkel zwischen den Kopf- und Hybriddomänen von β8 verglichen mit mit dem Antikörper ohne chemische Glykosylierung in Kontakt gebrachtem β8 verkleinert wird.

3. Verfahren nach Anspruch 1 oder 2, wobei der rekombinante oder glykosylierte Antikörper ein SEQ ID NO: 16 umfassendes Epitop bindet.

4. Verfahren nach einem der Ansprüche 1-3, wobei durch den rekombinanten oder glykosylierten Antikörper nach Bindung von β8 der Winkel zwischen den Kopf- und Hybriddomänen von β8 um wenigstens 5° verglichen mit nicht mit dem Antikörper in Kontakt gebrachtem β8 verkleinert wird.

5. Verfahren nach einem der Ansprüche 1-4, wobei die Aminosäure, die glykosyliert wird, Aminosäureposition 10 in einer von SEQ ID NO: 1-6 entspricht.

## Revendications

1. Procédé de production d'un anticorps recombinant qui inhibe la libération d'un peptide de TGFβ mature, actif, comprenant les étapes consistant à :
identifier un anticorps qui se lie à un épitope dans les domaines de tête et/ou hybride d'un β8 ;
modifier, de manière recombinante, la séquence de CDR2 d'une chaîne lourde pour introduire un site de glycosylation ; et
exprimer l'anticorps, l'anticorps provoquant, lors de la liaison au β8, un changement de conformation qui réduit l'angle entre les domaines de tête et hybride du β8, en comparaison avec un β8 n'ayant pas été mis en contact avec l'anticorps.

2. Procédé de production d'un anticorps glycosylé qui inhibe la libération d'un peptide de TGFβ mature, actif, comprenant les étapes consistant à :
identifier un anticorps qui se lie à un épitope dans les domaines de tête et/ou hybride d'un β8, et qui comprend un acide aminé qui peut être glycosylé dans la séquence de CDR2 d'une chaîne lourde ;
exprimer l'anticorps ; et
glycosyler chimiquement l'acide aminé, dans lequel l'anticorps provoque, lors de la liaison au β8, un changement de conformation qui réduit l'angle entre les domaines de tête et hybride du β8, en comparaison avec un β8 ayant été mis en contact avec l'anticorps sans glycosylation chimique.

3. Procédé des revendications 1 ou 2, dans lequel l'anticorps recombinant ou glycosylé se lie à un épitope comprenant la SEQ ID n° : 16.

4. Procédé de l'une quelconque des revendications 1 à 3, dans lequel, lors de la liaison au β8, l'anticorps recombinant ou glycosylé réduit d'au moins 5° l'angle entre les domaines de tête et hybride du β8, en comparaison avec un β8 n'ayant pas été mis en contact avec l'anticorps.

5. Procédé de l'une quelconque des revendications 1 à 4, dans lequel l'acide aminé qui est glycosylé correspond à un acide aminé en position 10 dans l'une quelconque des SEQ ID n° : 1 à 6.
